# EUROPEAN PATENT SPECIFICATION

(11) **EP 0 347 899 B1**
(45) Date of publication and mention of the grant of the patent: **30.08.1995**
(21) Application number: 89111364.9
(22) Date of filing: 22.06.1989
(51) Int. Cl.: A61K 35/54

(54) **Pharmaceutical compositions on egg shell basis and their preparation and use**
Pharmazeutische Zusammensetzungen mit Eierschalenbestandteilen und ihre Herstellung und Verwendung
Compositions pharmaceutiques à base de coquille d'oeuf et leur préparation et utilisation

(30) Priority: 24.06.1988 CS 4454/88
(43) Date of publication of application: 27.12.1989
(73) Proprietor: BIOMIN, akciová spolocnost', 919 43 Cifer (CS)
(72) Inventor: Michalek, Karol, Dr., Trnava (CS)
(74) Representative: Beetz & Partner Patentanwälte

(56) References cited:
- US-A- 3 558 771
- US-A- 5 045 323
- PATENT ABSTRACTS OF JAPAN, vol. 8, no. 260 (C-254)[169], 29th November 1984; & JP-A-59 137 415
- CHEMICAL ABSTRACTS, vol. 76, no. 20, 15th May 1972, page 324, abstract no. 117501z, Columbus, Ohio, US

## Description

The invention relates to pharmaceutical compositions on the basis of egg shells, particularly chicken egg shells, methods for their preparation, and their medical and pharmaceutical use.

The main elements contained in the compounds of egg shells are calcium, phosphorus, magnesium and a number of trace elements, in biogenous bonding. In addition thereto, egg shells contain constituents which are not comprised in the present pharmaceutical compositions for the treatment of mineral deficiency diseases.

At present, mostly inorganic raw materials are used for the preparation of different medicaments in this respect in the pharmaceutical industry. Medicaments prepared from inorganic raw materials, however, either do not comprise certain elements or contain these elements only in a form which does not enable their full physiological utilization. Actually, there is no really effective medicament available which could be applied for the treatment of certain diseases, particularly of osteoporosis, and coxarthrosis, which has been prepared on the basis of organically/biologically transformed and synthetized mineral compounds and of trace elements with proteinaceous bonding.

JP-A-59-137 415 discloses a method for obtaining a calcium agent from egg shells, which comprises the steps of separating the shells from eggs, removing residues by washing with water, drying the egg shells with hot air of a temperature of < 80 °C, and crushing and sieving with a 300 mesh sieve to obtain a particle size of about 40 »m. According to this document the egg shell material must not be heated to a temperature above 80 °C to prevent thermal degradation of mineral compounds, and to allow living active microorganisms to attack pathogenic bacteria and promote the action of the cells. Furthermore, the egg shell membrane is not removed in this prior art process.

It is the object of the present invention to provide novel pharmaceutical compositions on the basis of egg shells, particularly chicken egg shells, which can be effectivly utilized for medical purposes for the treatment of certain diseases, particularly mineral deficiency diseases, in particular with respect to living tissues, which can be fully utilized by the treated organism and also comprise certain elements in proteinaceous bonding, and to provide a method for their preparation and their pharmaceutical use.

The above object is achieved according to independent claims 1, 6 and 10. The dependent claims relate to preferred embodiments.

The pharmaceutical compositions according to the present invention may be obtained by the following process:
(A) emptying eggs, preferably chicken eggs,
(B) removing residues of egg yolk and white of egg, ballast constituents, contaminants and the membrane sticking to the interior egg shell surface, and purifying the egg shells,
(C) drying the egg shells with hot air,
(D) crushing and grinding the egg shells to a powder having a particle size of ≦ 150 »m, and preferably of 10 to 80 »m,
   and
(E) sterilizing the egg shell powder at a temperature of 120 °C to devitalize pathogenic and conditionally pathogenic microorganisms.

According to a preferred embodiment, removal of residues of egg yolk and white of egg and other constituents and contaminants including the membranes sticking to the interior surface of the egg shells is carried out by mechanical treatment and preferably by centrifugation; the subsequent purification is preferably carried out by washing with pure water and removing the water in step B, preferably by centrifugation.

According to another preferred embodiment, the drying of the egg shell material in step C is carried out with hot air of a temperature of ≦ 150 and preferably of 60 to 120 °C.

In accordance with another preferred embodiment, the crushed and ground egg shell material is sieved for obtaining particles having a diameter within a pre-determined particle size range.

The pharmaceutical compositions according to the present invention are capable to fully balance deficient biogenous mineral compounds and trace elements in the treatment of a number of diseases, particularly deficiency diseases, such as osteopathia and chondropathia, and other diseases of bones and joints where remineralization is required, as osteoporosis, and coxarthrosis., and where an increased speed of callus formation is required. Actually used preparations for the treatment of similiar diseases are based merely on inorganic compounds and thus are generally only little effective, and, particularly for geriatric purposes, involve also a considerable risk of lethal effects in gerontological applications.

So far egg shells have already been used for curing certain diseases, their application has been based rather on intuition without provision of a compound having stable, standardized properties, and without determination of their specific effectiveness and activity based on objective biological knowledge and pharmaceutical methods.

The speed and efficiency of metabolization of the pharmaceutical compositions according to the present invention can be checked by pharmacokinetic methods and by X-ray and particularly densitometric investigations of the conditions of the whole skeleton or of local areas, e.g. fractures, and of changes of the conditions of joints, spine and/or different bone tissues, in addition to the determination of a subjective improvement of the health condition of the patients.

The pharmaceutical compositions according to the present invention proved particularly effective for geriatry where currently used medicaments have not been effective or exhibited unfavourable side effects, or have not been properly tolerated by the body.

The pharmaceutical compositions according to the invention may be used in the form of the powder as obtained according to the above mentioned preparation method, the application of the product in the form of a powder being most effective.

Alternatively, the pharmaceutical compositions may be transformed into other galenic forms, e.g. by introducing other active ingredients and/or usual carriers, additives and/or excipients.

The method for preparing the compositions according to the invention from egg shells will be described in the following with more details.

After removal of egg yolk and white from eggs, the egg shells are first mechanically treated, preferably in a centrifugal separator, in order to remove any remaining egg yolk or white of egg; the centrifuged shells are then washed in water to remove residues and the membrane film sticking to the internal surface of the egg shells and for removing other contaminants; the material is subsequently rinsed with pure water whereafter the water is removed, advantageously by a centrifugal separator. The obtained product is then dried with hot air of a temperature of up to 150 °C, advantageously of a temperature of 60 to 120 °C, with following crushing and grinding to a fine powder of a particle size of up to 150 »m and advantageously between 10 and 80 »m. The product may then be sieved and is subsequently sterilized in order to devitalize pathogenic and conditionally pathogenic microorganisms. This sterilization should be carried out for about 1 h at 120 °C. The obtained final product may then be checked for possible traces of microorganisms.

The thus obtained powder comprises substantially the following biologically bonded elements:

| | |
|---|---|
| Calcium | 30,0 to 40,0 g/100 g of powder |
| magnesium | 0,35 to 0,5 g/100 g, |
| phosphorus | 0,08 g/100g, |
| carbonates | 46,0 to 60,0 g/100 g, and |
| nitrogen | 0,5 to 0,6 g/100 g, |

and other biogenous elements, all in biological bonding.

The pharmaceutical compositions of the present invention may be advantageously administered per os. Nevertheless, also other usual galenic forms may be used for administration.

The pharmaceutical compositions according to the invention can be advantageously utilized in medical therapy, particularly in pediatry, traumatology, geriatry, orthopedy, stomatology, puerperium, oncology and pneumopathy.

## Claims

1. Pharmaceutical compositions on the basis of egg shells,
obtainable by
(A) emptying eggs, preferably chicken eggs,
(B) removing residues of egg yolk and white of egg, ballast constituents, contaminants and the membrane sticking to the interior egg shell surface, and purifying the egg shells,
(C) drying the egg shells with hot air,
(D) crushing and grinding the egg shells to a powder having a particle size of ≦ 150 »m, and preferably of 10 to 80 »m,
and
(E) sterilizing the egg shell powder at a temperature of 120 °C to devitalize pathogenic and conditionally pathogenic microorganisms.

2. Pharmaceutical compositions according to claim 1, obtainable by mechanical treatment, preferably by centrifugation, subsequent washing with pure water, and removing the water, preferably by centrifugation, in step B.

3. Pharmaceutical compositions according to claim 1 or 2, obtainable by drying the egg shells in step C with hot air of a temperature of ≦ 150 °C and preferably of 60 to 120 °C.

4. Pharmaceutical compositions according to one of claims 1 to 3, obtainable by sieving the ground egg shell material in step D.

5. Pharmaceutical compositions according to one of claims 1 to 6, comprising, in addition to trace elements and other biogenous elements,
| | |
|---|---|
| calcium | 30 to 40 g/100 g of powder, |
| magnesium | 0,35 to 0,5 g/100 g, |
| phosphorus | 0,08 g/100 g, |
| carbonates | 46,0 to 60,0 g/100 g, and |
| nitrogen | 0,5 to 0,6 g/100 g, |
in biological bonding.

6. A method for preparing the pharmaceutical compositions according to one of claims 1 to 5,
comprising the following steps:
(A) emptying eggs, preferably chicken eggs,
(B) removing residues of egg yolk and white of egg, ballast constituents, contaminants and the membrane sticking to the interior egg shell surface, and purifying the egg shells,
(C) drying the egg shells with hot air,
(D) crushing and grinding the egg shells to a powder having a particle size of ≦ 150 »m, and preferably of 10 to 80 »m,
and
(E) sterilizing the egg shell powder at a temperature of 120 °C to devitalize pathogenic and conditionally pathogenic microorganisms.

7. The method according to claim 6, characterized by mechanical treatment, preferably by centrifuging, subsequent washing with pure water, and removing the water, preferably by centrifugation, in step B.

8. The method according to claim 6 or 7, characterized by drying the egg shells in step C with hot air of a temperature of ≦ 150 °C and preferably of 60 to 120 °C.

9. The method according to one of claims 6 to 8, characterized by sieving the ground egg shell material in step D.

10. Use of the pharmaceutical compositions according to one of claims 1 to 5 for the manufacture of pharmaceutical compositions for the treatment of mineral deficiencies, particularly in joint and bone tissues, and particularly of osteoporosis and coxarthrosis.

## Patentansprüche

1. Pharmazeutische Zusammensetzungen auf der Basis von Eierschalen, die erhalten werden können durch
(A) Leeren von Eiern, vorzugsweise Hühnereiern,
(B) Entfernen der Reste von Eigelb und von Eiklar, von Ballastbestandteilen, Verunreinigungen und der Membran, die an der inneren Eierschalenoberfläche anhaften, und Reinigen der Eierschalen,
(C) Trocknen der Eierschalen mit heißer Luft,
(D) Zerkleinern und Mahlen der Eierschalen zu einem Pulver mit einer Partikelgröße von ≦ 150 »m und vorzugsweise von 10 bis 80 »m,
und
(E) Sterilisieren des Eierschalenpulvers bei einer Temperatur von 120°C, um pathogene und bedingt pathogene Mikroorganismen abzutöten.

2. Pharmazeutische Zusammensetzungen nach Anspruch 1, die im Schritt B durch mechanische Behandlung, vorzugsweise durch Zentrifugieren, anschließendes Waschen mit reinem Wasser und Entfernen des Wassers, vorzugsweise durch Zentrifugieren erhalten werden können.

3. Pharmazeutische Zusammensetzungen nach Anspruch 1 oder 2, die im Schritt C durch Trocknen der Eierschalen mit heißer Luft mit einer Temperatur von ≦ 150°C und vorzugsweise von 60 bis 120°C erhalten werden können.

4. Pharmazeutische Zusammensetzungen nach einem der Ansprüche 1 bis 3, die im Schritt D durch Sieben des gemahlenen Eierschalenmaterials erhalten werden können.

5. Pharmazeutische Zusammensetzungen nach einem der Ansprüche 1 bis 6, die zusätzlich zu den Spurenelementen und anderen biogenen Elementen enthalten:
| | |
|---|---|
| Calcium | 30 bis 40 g/100 g des Pulvers, |
| Magnesium | 0,35 bis 0,5 g/100 g, |
| Phosphor | 0,08 g/100 g, |
| Carbonate | 46,0 bis 60,0 g/100 g, und |
| Stickstoff | 0,5 bis 0,6 g/100 g |
in biologischer Bindung.

6. Verfahren zum Herstellen der pharmazeutischen Zusammensetzungen nach einem der Ansprüche 1-5, mit den folgenden Schritten:
(A) Leeren von Eiern, vorzugsweise Hühnereiern,
(B) Entfernen der Reste von Eigelb und von Eiklar, von Ballastbestandteilen, von Verunreinigungen und der Membran, die an der inneren Eierschalenoberfläche anhaften, und Reinigen der Eierschalen,
(C) Trocknen der Eierschalen mit heißer Luft,
(D) Zerkleinern und Mahlen der Eierschalen zu einem Pulver mit einer Partikelgröße von ≦ 150 »m und vorzugsweise 10 bis 80 »m, und
(E) Sterilisieren des Eierschalenpulvers bei einer Temperatur von 120°C, um pathogene und bedingt pathogene Mikroorganismen abzutöten.

7. Verfahren nach Anspruch 6, gekennzeichnet durch mechanische Behandlung, vorzugsweise durch Zentrifugieren, anschließendes Waschen mit reinem Wasser und Entfernen des Wassers, vorzugsweise durch Zentrifugieren, im Schritt B.

8. Verfahren nach Anspruch 6 oder 7, gekennzeichnet durch Trocknen der Eierschalen im Schritt C mit heißer Luft mit einer Temperatur von ≦ 150°C und vorzugsweise 60 bis 120°C.

9. Verfahren nach einem der Ansprüche 6 bis 8, gekennzeichnet durch Sieben des gemahlenen Eierschalenmaterials im Schritt D.

10. Verwendung der pharmazeutischen Zusammensetzungen nach einem der Ansprüche 1 bis 5 für die Herstellung von pharmazeutischen Zusammensetzungen für die Behandlung von Mineralmangel, insbesondere in Gelenk- oder Knochengeweben und insbesondere der Osteoporose und der Koxarthrose.

## Revendications

1. Compositions pharmaceutiques à base de coquilles d'oeufs, pouvant être obtenues par le processus consistant à :
(A) vider les oeufs, de préférence des oeufs de poule,
(B) retirer les résidus de jaune d'oeuf et de blanc d'oeuf, les éléments constitutifs du lest, les produits de contamination et la membrane collant à la surface intérieure de la coquille d'oeuf, et purifier les coquilles d'oeufs,
(C) faire sécher les coquilles d'oeufs par de l'air chaud,
(D) écraser et moudre les coquilles d'oeufs pour obtenir une poudre présentant une taille de particules inférieure ou égale à 150 »m, et de préférence de 10 à 80 »m,
et
(E) stériliser la poudre de coquilles d'oeufs à une température de 120°C pour dévitaliser les microorganismes pathogènes et conditionnellement pathogènes.

2. Compositions pharmaceutiques selon la revendication 1, pouvant être obtenues par un traitement mécanique, de préférence par centrifugation, puis par lavage à l'eau pure et extraction de l'eau, de préférence par centrifugation, dans l'étape B.

3. Compositions pharmaceutiques selon la revendication 1 ou 2, pouvant être obtenues en faisant sécher les coquilles d'oeufs dans l'étape C par de l'air chaud à une température inférieure ou égale à 150°C, et de préférence de 60° à 120°C.

4. Compositions pharmaceutiques selon l'une des revendications 1 à 3, pouvant être obtenues en tamisant le matériau de coquilles d'oeufs moulues dans l'étape D.

5. Compositions pharmaceutiques selon l'une des revendications 1 à 4, comprenant, en plus d'oligo-éléments et d'autres éléments biogènes :
| | |
|---|---|
| Calcium | 30,0 à 40,0 g/100 g de poudre |
| Magnésium | 0,35 à 0,5 g/100 g, |
| Phosphore | 0,08 g/100 g, |
| Carbonates | 46,0 à 60,0 g/100 g, et |
| Azote | 0,5 à 0,6 g/100 g, |
en liaison biologique.

6. Procédé de préparation des compositions pharmaceutiques selon l'une des revendications 1 à 5,
comprenant les étapes suivantes consistant à :
(A) vider les oeufs, de préférence des oeufs de poule,
(B) retirer les résidus de jaune d'oeuf et de blanc d'oeuf, les éléments constitutifs du lest, les produits de contamination et la membrane collant à la surface intérieure de la coquille d'oeuf, et purifier les coquilles d'oeufs,
(C) faire sécher les coquilles d'oeufs par de l'air chaud,
(D) écraser et moudre les coquilles d'oeufs pour obtenir une poudre présentant une taille de particules inférieure ou égale à 150 »m, et de préférence de 10 à 80 »m,
et
(E) stériliser la poudre de coquilles d'oeufs à une température de 120°C pour dévitaliser les micro-organismes pathogènes et conditionnellement pathogènes.

7. Procédé selon la revendication 6, caractérisé par un traitement mécanique, de préférence par centrifugation, après lavage à l'eau pure et extraction de l'eau, de préférence par centrifugation, dans l'étape B.

8. Procédé selon la revendication 6 ou 7, caractérisé par le séchage des coquilles d'oeufs dans l'étape C en utilisant de l'air chaud à une température inférieure ou égale à 150°C, et de préférence de 60° à 120°C.

9. Procédé selon l'une des revendications 6 à 8, caractérisé par le tamisage du matériau de coquilles d'oeufs moulues dans l'étape D.

10. Utilisation des compositions pharmaceutiques selon l'une des revendications 1 à 5, pour la fabrication de compositions pharmaceutiques destinées au traitement de carences minérales, en particulier dans les articulations et les tissus osseux, et plus particulièrement pour le traitement de l'ostéoporose et de la coxarthrose.
